# EUROPÄISCHE PATENTSCHRIFT

(11) **EP 0 920 283 B1**
(45) Veröffentlichungstag und Bekanntmachung des Hinweises auf die Patenterteilung: **07.03.2001**
(21) Anmeldenummer: 97934436.3
(22) Anmeldetag: 21.07.1997
(51) Int. Cl.: A61B 18/12

(54) **INSTRUMENT ZUM SCHNEIDEN VON GEWEBE MIT HF-STROM**
INSTRUMENT FOR CUTTING TISSUE USING HIGH-FREQUENCY CURRENT
INSTRUMENT POUR DECOUPER DES TISSUS A L'AIDE DE COURANT HAUTE FREQUENCE

(30) Priorität: 19.07.1996 DE 19629278
(43) Veröffentlichungstag der Anmeldung: 09.06.1999
(73) Patentinhaber: Karl Storz GmbH & Co. KG, 78532 Tuttlingen (DE)
(72) Erfinder: MONNIER, Philippe, CH-1009 Pully (CH)
(74) Vertreter: Münich, Wilhelm, Dr.
(86) Internationale Anmeldenummer: DE9701526
(87) Internationale Veröffentlichungsnummer: WO9803122

(56) Entgegenhaltungen:
- DE-A- 19 501 258
- US-A- 5 423 813
- US-A- 5 527 332

## Beschreibung

### Technisches Gebiet

Die Erfindung bezieht sich auf ein Instrument zum Schneiden von Gewebe gemäß dem Oberbegriff des Patentanspruchs 1.

### Stand der Technik

Ein Instrument zum Schneiden von Gewebe ist aus der US-PS 5,423,830 bekannt. Dieses Instrument, das ausdrücklich zum Entfernen von Polypen gedacht ist, weist eine HF-Schlinge auf, die über den Polypen geschoben wird. Anschließend wird der Polyp in eine Saugglocke eingesaugt und der Fuß des Polypen mit der Schlinge durchtrennt.

Das bekannte gattungsgemäße Instrument eignet sich damit nicht zum streifenförmigen Abschneiden insbesondere von Schleimhaut, wie dies beispielsweise bei bestimmten Behandlungsvorgängen von länglichen Organen, wie z.B. des Oesophagus erforderlich ist.

Ein Instrument geringfügig abweichender Gattung ist aus der DE 44 29 478 C1 bekannt. Dieses Instrument weist neben einer mit Hochfrequenz betriebenen Schneideeinrichtung ein Extraktionsrohr auf, über das abgeschnittene Gewebeteile abgesaugt werden können. Auch dieses Instrument eignet sich nicht zum Abschneiden von Streifen, beispielsweise der Schleimhaut.

Aus der US-A 5,423,813, von der bei der Formulierung des Oberbegriffs des Patentanspruch 1 ausgegangen worden ist, ist ein Resektoskop und eine Elektrodenanordnung bekannt, bei der sich die Elektrodenanordnung, die u.a. eine Schneid-Schlinge aufweisen kann, in dem Instrumentenschaft befindet. Zum Schneiden kann die Schlinge über das distale Ende des Instrumentenschafts hinausgezogen werden. Um das abgeschnittene Gewebe aus dem Körper entnehmen zu können, wird dieses in den Instrumentenschaft hineingesaugt. Hierzu ist eine Saug- bzw. Unterdruckeinrichtung vorgesehen. Das aus diesem Dokument bekannte Instrument zum Schneiden von Gewebe dient im wesentlichen zum Abschneiden von Wucherungen etc., wobei Voraussetzung ist, dass das abzuschneidende Gewebe gegenüber dem restlichen Gewebe "vorsteht". Dieses Instrument ist nicht zum flächigen Abtragen von Gewebe in schichtweiser Form geeignet.

### Darstellung der Erfindung

Der Erfindung liegt die Aufgabe zugrunde, ein Instrument zum Schneiden von Gewebe mit HF-Strom anzugeben, mit dem das Gewebe mit einer bestimmten Dicke streifenförmig geschnitten werden kann.

Eine erfindungsgemäße Lösung dieser Aufgabe ist im Patentanspruch 1 angegeben. Weiterbildungen der Erfindung sind Gegenstand der Ansprüche 2 ff.

Erfindungsgemäß weist der Instrumentenkörper seitlich in der Mantelfläche an einem mit dem Gewebe in Berührung kommenden Bereich ein Ansaugelement auf, an das mittels der Saugeinrichtung, wie beispielsweise einer Pumpe derart Unterdruck angelegt wird, daß Gewebe an den Instrumentenkörper insbesondere im Bereich des Ansaugelements angesaugt wird. Im Gegensatz zum Stand der Technik soll dabei das Gewebe nicht in den Instrumentenkörper hineingesaugt werden, sondern durch die Saugwirkung lediglich an dem Instrumentenkörper "anhaften". Zum Abschneiden von streifenförmigen Gewebeteilen mit im wesentlichen konstanter Dicke wird wenigstens ein Teil der Elektrodenanordnung derart parallel zum Ansaugelement bewegt, daß das an den Instrumentenkörper angesaugte Gewebe zwischen Ansaugelement und dem bewegten Teil der Elektrodenanordnung abgetragen wird. Unabhängig davon, ob eine monopolare oder eine bipolare Elektrodenanordnung verwendet wird hat der bewegbare Teil der Elektrodenanordnung die Form einer an sich bekannten HF-Schneid-Schlinge. Die genaue Form der Schlinge ist der Außenkontur des Ansaugelements angepaßt.

Die Schlinge befindet sich in einem vorgegebenen Abstand in Richtung senkrecht zur Oberfläche des Ansaugelements, der die Dicke der abgeschnittenen Gewebestreifen bestimmt (Anspruch 6).

Zum Schneiden von Gewebe ist die Schlinge dabei bevorzugt vom distalen Ende in Richtung auf das proximale Ende bewegbar, da bei einem "Arbeiten auf Zuge die Kraftübertragung besser zu dosieren ist als bei einem Arbeiten auf "Druck" (Anspruch 7).

Für das Ansaugelement ist bevorzugt die im Anspruch 2 angegebene Weiterbildung vorgesehen, gemäß der das Ansaugelement eine bevorzugt zylindrische Außenmantelfläche aufweist, in der eine Lochanordnung mit wenigstens einem Loch vorgesehen ist, oder die permeabel ausgebildet ist. An die zylindrische Außenmantelfläche wird von innen Unterdruck angelegt. Im Falle der Verwendung einer Lochanordnung ist es bevorzugt, wenn diese eine Mehrzahl von Löchern mit relativ kleinem Durchmesser aufweist (Anspruch 3), da sich hierdurch eine große Ansaugwirkung ergibt, ohne daß die Gefahr bestünde, daß Gewebe in den Instrumentenkörper "hineingesaugt" wird.

Die HF-Elektrodenanordnung im Instrument kann sowohl eine monopolare Elektrode, bei der die Gegenelektrode beabstandet am Körper angebracht wird, als auch eine bipolare Elektrodenanordnung sein (Ansprüche 4 bzw. 5).

Wenn das Instrument eine monopolare Elektrode aufweist, wird diese von dem beweglichen Teil gebildet. Die Neutralelektrode wird in bekannter Weise an der Körperoberfläche angebracht.

Im Falle einer bipolaren Elektrodenanordnung wird die eine Elektrode von dem bewegten Teil der Elektrodenanordnung und die andere Elektrode (bevorzugt) von der zylindrischen Außenmantelfläche gebildet.

Im Anspruch 8 ist eine besondere Ausgestaltung des erfindungsgemäßen Instruments angegeben, bei der im wesentlichen unveränderte bzw. nur geringfügig modifizierte "Baukastenteile" bekannter Endoskop- und HF-Resektionssysteme verwendet werden können: Bei dieser Ausgestaltung weist der Instrumentenkörper einen ersten Hohlschaft auf, der im wesentlichen bekannten Endoskop-Schäften entspricht. Dieser Hohlschaft ist derart modifiziert, daß er das seitliche Ansaugelement trägt. An seinem proximalen Ende ist ein an sich bekannter Anschluß für die Saugeinrichtung, wie beispielsweise eine Saugpumpe vorgesehen, die eine Wasserstrahlpumpe, eine Kolbenpumpe, eine Rotationspumpe oder dgl. sein kann.

Eine besonders kostengünstige Herstellung und darüber hinaus eine leichte Sterilisier- und Reinigbarkeit des erfindungsgemäßen Instruments erhält man dadurch, daß die seitliche Lochanordnung bzw. der permeable Bereich in der Außenwand eines Hohlkörpers vorgesehen ist, der in eine Ausnehmung in dem ersten Schaft eingesetzt ist (Anspruch 9). Gemäß Anspruch 10 kann der Hohlkörper insbesondere dann, wenn die Elektrodenanordnung eine monopolare Elektrode ist, aus einem elektrisch isolierenden Material, wie einem Keramikmaterial, einem Glasmaterial, Quarz oder einem Kunststoffmaterial bestehen (Anspruch 11).

Dabei ist es bevorzugt, wenn der Hohlkörper transparent ist (Anspruch 12), da dann eine Beobachtung mittels eines in den ersten Schaft eingesetzten Endoskops (Anspruch 18) durch den transparenten Hohlkörper hindurch möglich ist. Im Falle der Verwendung relativ größer Löcher ist es selbstverständlich möglich, den Hohlkörper auch aus einem nichttransparenten Material zu fertigen und die Beobachtung durch die Löcher vorzunehmen.

Anstelle einer Seitblickendoskopoptik (Anspruch 19) kann auch eine Retrooptik verwendet werden, die über das distale Ende des ersten Schafts vorsteht (Anspruch 20). In dem Falle, daß die Endoskopoptik in dem Kanal des ersten Schafts geführt wird, durch den auch abgesaugt wird, ist ggf. eine Dichtung am distalen Ende vorzusehen.

In jedem Falle ist es gemäß Anspruch 21 bevorzugt, wenn die Endoskopoptik axial verschiebbar ist.

In den Ansprüchen 13 und 14 sind weitere Ausgestaltungen des Hohlkörpers beschrieben, durch die weiter die Reinigbarkeit und Sterilisierbarkeit des Instruments verbessert wird.

Die Ansprüche 15 bis 17 ermöglichen bei kleinem Aufbau und insbesondere geringem benötigtem Lumen eine einfache Verschiebung der bewegbaren Elektrode.

### Kurze Beschreibung der Zeichnung

Die Erfindung wird nachstehend anhand eines Ausführungsbeispiels unter Bezugnahme auf die Zeichnung näher beschrieben, in der zeigen:
- Fig. 1a bis d: eine "Explosionszeichnung", und
- Fig. 2a und b: Seitenansichten des in Fig. 1 dargestellten Instruments in unterschiedlichen Zusammenbaustufen.

### Beschreibung eines Ausführungsbeispiels

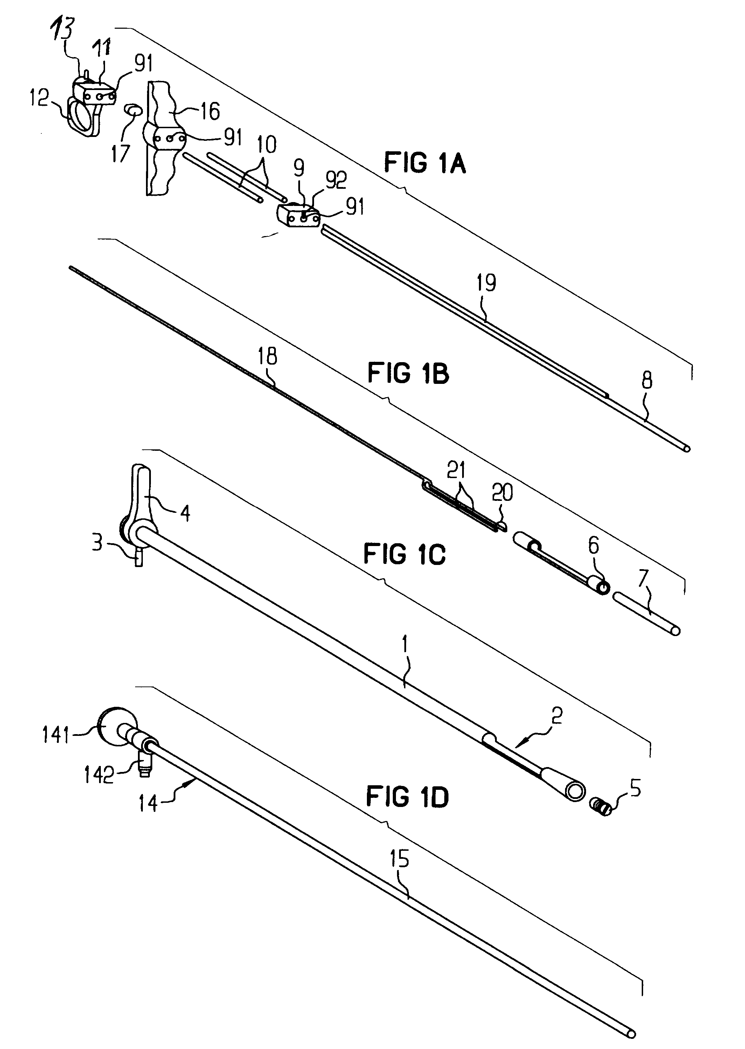

Zunächst soll der Grundaufbau des erfindungsgemäßen Instruments unter Bezugnahme auf die Fig. 1a bis 1d erläutert werden, auf die im übrigen bezüglich der Ausgestaltung aller - gegebenenfalls auch erfindungswesentlicher und in der Beschreibung nicht ausdrücklich genannter - Details ausdrücklich verwiesen wird.

Das erfindungsgemäße Instrument zum Schneiden von Gewebe insbesondere in einem Hohlraum eines menschlichen oder tierischen Körpers weist einen ersten Hohlschaft 1 (Fig. 1c) auf, der im Bereich seines distalen Endes eine seitliche Ausnehmung 2 in seiner zylindrischen Umfangs- bzw. Mantelfläche hat. Am proximalen Ende des Schafts 1 ist ein Anschluß 3 für eine Saugeinrichtung, wie eine Saugpumpe, und eine Handhabe 4 vorgesehen. In die Stirnfläche des distalen Endes des Hohlschaft ist eine Schraube 5 eingesetzt, deren Funktion weiter unten noch erläutert wird.

In den ersten Hohlschaft 1 ist ein Trägerelement 6 (Fig. 1b) eingesetzt, das einen Hohlkörper 7 derart aufnimmt, daß ein Teil der zylindrischen Mantelfläche des Hohlkörpers aufgrund der seitlichen Ausnehmung 2 des Hohlschafts 1 "frei liegt". Der Hohlkörper 7 weist eine Mehrzahl von Löchern in seiner zylindrischen Mantelflächefläche auf, an die mittels der nicht dargestellten und mit dem Anschluß 3 verbundenen Saugpumpe Unterdruck anlegbar ist.

Bei dem gezeigten Ausführungsbeispiel ist das Trägerelement 6 mit dem Schaft 1 mittels der Schraube 5 durch die distale Stirnfläche des Schafts verschraubt.

In den ersten Hohlschaft 1 ist ein zweiter Hohlschaft 8 (Fig. 1a) eingesetzt, dessen distale Stirnfläche an die dem proximalen Ende zugekehrte Stirnfläche des Trägerelements 6 angrenzt. Bei dem gezeigten Ausführungsbeispiel sind der zweite Hohlschaft 8 und das Trägerelement 6 nicht miteinander verbunden, selbstverständlich ist es aber möglich, alternativ zur Verschraubung des Trägerelements 6 durch die distale Stirnfläche des ersten Hohlschafts 1 das Trägerelement mit den zweiten Hohlschaft 8 zu verbinden. Am proximalen Ende des zweiten Hohlschafts 8 ist ein Verbindungsblock 9 vorgesehen, der eine Durchgangsbohrung 91, die mit dem zentralen Kanal des Hohlschafts 8 fluchtet, und eine weitere Durchgangsbohrung 92 aufweist, deren Funktion noch erläutert werden wird.

Auf der proximalen Seite des Verbindungsblocks 9 sind zwei Führungsstangen 10 vorgesehen, deren anderes Ende in einem weiteren Block 11 gelagert ist. An dem weiteren Block 11 sind ein erster Handgriff 12 - bei dem gezeigten Ausführungsbeispiel ein Daumenring - und eine Kupplung 13 für Instrumente, wie beispielsweise eine an sich bekannte Bajonettkupplung, vorgesehen. Der weitere Block 11 weist ebenfalls eine Durchgangsbohrung 91 auf, die mit dem zentralen Kanal des zweiten Schafts 8 fluchtet.

Damit kann in die Kupplung 13 ein herkömmliches Endoskop 14 (Fig. ld) mit Okular 141 und Lichtleiteranschluß 142 derart eingesetzt werden, daß der Schaft 15 des Endoskops 14 die Durchgangsbohrungen 91 der Blöcke 9 und 11 sowie den zweiten Hohlschaft 8 und die Elemente 6 und 7, die letzteren zumindest teilweise - durchsetzt.

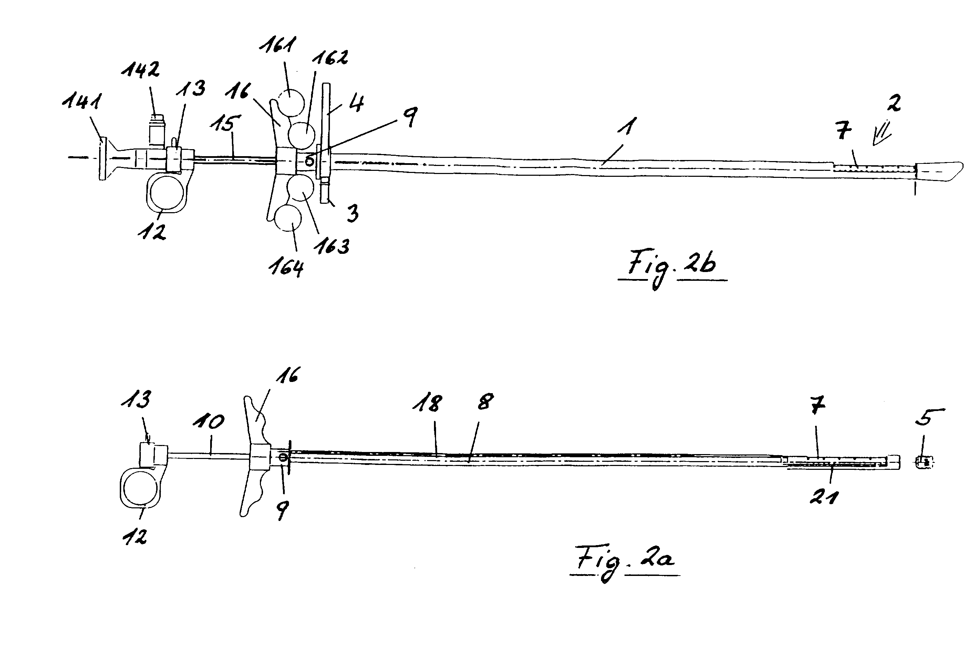

Auf den beiden Führungsstanqen 10 ist ein zweiter Handgriff 16 verschiebbar gelagert, der bei dem gezeigten Ausführungsbeispiel mit Anlagen für - wie Fig. 2b zeigt - den Zeigefinger 161, den Mittelfinger 162, den Ringfinger 163 und den kleinen Finger 164 versehen ist.

Auf der proximalen Seite des zweiten Handgriffs 16 ist ein Anschluß 17 für den einen Pol eines nicht gezeigten HF-Chiruriegeräts vorgesehen, der durch die Bohrung 92 mit der Stange 18 verbunden ist. Der andere Pol dieses HF-Chirurgiegeräts ist bei dem gezeigten Ausführungsbeispiel mit einer nicht gezeigten Neutralelektrode verbunden, die in bekannter Weise an der Körperoberfläche des behandelten menschlichen oder tierischen Körpers angebracht ist.

Ein stabförmiges Teil 18 (Fig. 1b), das in einer Führung 19 (Fig. 1a) auf der Oberfläche des zweiten Hohlschafts 8 geführt ist, ist durch den Handgriff 16 mit dem Anschluß 17 verbunden. Mit dem distalen Ende des Teils 18 ist eine HF-Schlinge 20 über Verbindungen 21 verbunden. Der stabförmige Teil 18 dient sowohl als Stromzuführung zu der HF-Schlinge 20 als auch als Element zur Übertragung einer Zugkraft bzw. Druckkraft.

Fig. 2a zeigt das Ausführungsbeispiel eines erfindungsgemäßen Instruments bei weggelassenem ersten Hohlschaft 1 (Außenschaft), so daß das Trägerelement 6 mit dem Hohlkörper 7, die distale angeordnete Schraube 5 sowie der zweite Hohlschaft 8 mit HF-Schlinge 20 zu sehen sind. Dabei sind gleiche Teile wie in Fig. 1 mit den selben Bezugszeichen versehen.

Fig. 2b zeigt das Ausführungsbeispiel mit aufgesetztem erstem Hohlschaft 1 (Außenschaft), wobei zusätzlich noch das Endoskop 14 in den zweiten Hohlschaft 8 eingesetzt ist. Ferner sind abstrahiert der Zeigefinger 161, der Mittelfinger 162, der Ringfinger 163 und der kleine Finger 164 anliegend an den Anlagen des zweiten Handgriffs 16 dargestellt.

Das erfindungsgemäße Instrument zum Schneiden von Gewebe mit HF-Strom arbeitet wie folgt:

Im ersten Schritt wird das Instrument in den Hohlraum des menschlichen Körpers eingesetzt und mit dem intrakorporalen Gewebe in Kontakt gebracht. Bei einer "nicht-endoskopischen" Anwendung wird natürlich das Instrument nur mit dem zun schneidenden "oberflächlichen" Gewebe in Kontakt gebracht. Die HF-Schlinge 20 befindet sich dabei in ihrer distalen Endposition.

Anschließend wird die Saugeinrichtung, also beispielsweise eine Pumpe eingeschaltet, so daß Unterdruck an den Hohlkörper 7 angelegt wird, der als Ansaugelement dient. Durch den Unterdruck wird das zu schneidende Gewebe an den Hohlkörper "angesaugt, so daß es an ihm "haftet".

Nunmehr wird das HF-Chirurgiegerät eingeschaltet, so daß bei einer monopolaren Elektrodenanordnung Strom zwischen der Schneidschlinge 20 und einer Neutralelektrode fließt. Bei Verwendung einer bipolaren Elektrodenanordnung fließt entsprechend der Strom zwischen der Schneidschlinge 20 und dem Instrumentenkörper, alos beispielsweise dem Hohlkörper 7.

Nach Einschalten des HF-Stroms wird die HF-Schneidschlinge 20 durch Zurückziehen des Handgriffs 16 parallel zur Oberfläche des Hohlkörpers 7 bewegt. Hierdurch wird das am Instrumentenkörper angesaugte Gewebe zwischen Ansaugelement und dem bewegten Teil der Elektrodenanordnung, also der Schlinge 20 abgetragen.

Vorstehend ist die Erfindung anhand eines Ausführungsbeispiels ohne Beschränkung des allgemeinen Erfindungsgedankens beschrieben worden, innerhalb dessen selbstverständlich die verschiedensten Abwandlungen möglich sind.

## Patentansprüche

1. Instrument zum Schneiden von Gewebe mit
- einem länglichen Instrumentenkörper (1), der in den menschlichen oder tierischen Körper einsetzbar ist,
- einer HF-Elektrodenanordnung (18,21;20), an die HF-Strom anlegbar ist, die in Richtung der Längsachse des Instrumentenkörpers verschiebbar ist, und die eine Schlinge (20) aufweist, und
- einer Saug- bzw. Unterdruckeinrichtung zum Anlegen von Unterdruck an ein Ansaugelement (7),
dadurch **gekennzeichnet**, daß das Ansaugelement (7) seitlich in der Mantelfläche des Instrumentenkörper (1) vorgesehen und derart ausgebildet ist, daß es das Gewebe an die Mantelfläche ansaugt, und
daß die sich an der Elektrodenanordnung befindende Schlinge (20) seitlich am Instrumentenkörper mit einem bestimmten Abstand von diesem angeordnet ist, und daß die Form der Schlinge (20) der Querschnittskontur der Mantelfäche angepaßt ist, so daß das an der Mantelfläche anliegende Gewebe durch die Verschiebung der Schlinge (20) in Richtung der Längsachse des Instrumentenkörpers schichtweise abtragbar ist.

2. Instrument nach Anspruch 1,
dadurch **gekennzeichnet,** daß die Mantelfläche zylindrisch ausgebildet ist, und
daß wenigstens ein Loch in der Mantelfläche oder wenigstens ein permeabler Bereich das Ansaugelement bilden.

3. Instrument nach Anspruch 2,
dadurch **gekennzeichnet,** daß eine Mehrzahl von Löchern vorgesehen sind.

4. Instrument nach einem der Ansprüche 1 bis 3,
dadurch **gekennzeichnet**, daß die HF-Elektrodenanordnung eine monopolare Elektrode (20) aufweist, die durch den bewegten Teil gebildet wird.

5. Instrument nach einem der Ansprüche 2 bis 3,
dadurch **gekennzeichnet**, daß die HF-Elektrodenanordnung eine bipolare Elektrodenanordnung ist, deren eine Elektrode von dem bewegten Teil und deren andere Elektrode von der zylindrischen Außenmantelfläche gebildet wird.

6. Instrument nach Anspruch 1 bis 5,
dadurch **gekennzeichnet**, dass die Schlinge (20) die Form einer an sich bekannten HF-Schneidschlinge besitzt und sich in einem vorgegebenen Abstand in Richtung senkrecht zur Oberfläche des Ansaugelements befindet, der die Dicke der abzuschneidenden Gewebestreifen bestimmt.

7. Instrument nach Anspruch 6,
dadurch **gekennzeichnet**, daß die Schlinge (20) zum Schneiden von Gewebe über die Außenmantelfläche in Richtung auf das proximale Ende hin bewegbar ist.

8. Instrument nach einem der Ansprüche 2 bis 6,
dadurch **gekennzeichnet**, daß der Instrumentenkörper einen ersten Hohlschaft (1) aufweist, der das seitliche Ansaugelement (7) und an seinem proximalen Ende einen Anschluß (3) für eine Saugeinrichtung, wie eine Saugpumpe aufweist.

9. Instrument nach Anspruch 8,
dadurch **gekennzeichnet**, daß die seitliche Lochanordnung oder der permeable Bereich in der Außenwand eines Hohlkörpers (7) vorgesehen ist, der in eine Ausnehmung (2) in dem ersten Schaft (1) eingesetzt ist.

10. Instrument nach Anspruch 9,
dadurch **gekennzeichnet**, daß der Hohlkörper (7) aus einem elektrisch isolierenden Material besteht.

11. Instrument nach Anspruch 10,
dadurch **gekennzeichnet**, daß das elektrisch isolierende Material ein Keramikmaterial, ein Glasmaterial, SiO₂ oder ein Kunststoffmaterial ist.

12. Instrument nach Anspruch 11,
dadurch **gekennzeichnet,** daß der Hohlkörper (7) transparent ist.

13. Instrument nach einem der Ansprüche 9 bis 12,
dadurch **gekennzeichnet**, daß der Hohlkörper (7) in ein Trägerelement (6) eingesetzt ist, mit dem zusammen er in den ersten Schaft (1) eingesetzt ist.

14. Instrument nach Anspruch 13,
dadurch **gekennzeichnet**, daß das Trägerelement (6) in dem ersten Schaft (1) mittels einer in die distale Stirnfläche des ersten Schafts eingesetzten Schraube (5) befestigt ist.

15. Instrument nach einem der Ansprüche 8 bis 14,
dadurch **gekennzeichnet**, daß die Zuleitung (18) für die Elektrodenanordnung (20) außerhalb der Achse in dem ersten Schaft (1) angeordnet und als Schub- bzw. Zugstange ausgebildet ist.

16. Instrument nach Anspruch 14,
dadurch **gekennzeichnet**, daß am proximalen Ende der Zugstange (18) ein Handgriff (12) angebracht ist.

17. Instrument nach einem der Ansprüche 8 bis 16,
dadurch **gekennzeichnet,** daß an dem ersten Schaft (1) eine Handhabe (4) angebracht ist.

18. Instrument nach einem der Ansprüche 8 bis 17,
dadurch **gekennzeichnet,** daß axial in den ersten Schaft (1) eine Endoskopoptik (14) eingesetzt ist.

19. Instrument nach Anspruch 18,
dadurch **gekennzeichnet**, daß die Endoskopoptik (14) eine Seitblickoptik ist, deren Blickfeld durch den transparenten Hohlkörper (7) hindurch gerichtet ist.

20. Instrument nach Anspruch 18,
dadurch **gekennzeichnet**, daß die Endoskopoptik (14) eine Retrooptik ist, und über das distale Ende des ersten Schaftes (1) vorsteht.

21. Instrument nach einem der Ansprüche 18 bis 20,
dadurch **gekennzeichnet**, daß die Endoskopoptik (14) axial positionierbar ist.

22. Instrument nach Anspruch 21,
dadurch **gekennzeichnet**, daß die Endoskopoptik (14) in einen zweiten Schaft (8) eingesetzt und mit diesem gemeinsam positionierbar ist.

23. Instrument nach Anspruch 22,
dadurch **gekennzeichnet**, daß an dem zweiten Schaft eine Handhabe (12), wie ein Daumenring angebracht ist.

## Claims

1. Instrument for cutting tissue, comprising
- an elongate instrument body (1) adapted to be introduced into the human or animal body,
- an HF electrode array (18, 21; 20) to which HF current is applicable and which is displaceable along the longitudinal axis of said instrument body and which comprises a loop (20), and
- a suction or vacuum means, respectively, for applying a vacuum to an aspiration element (7),
**characterised** in that said aspiration element (7) is provided laterally in the outside surface of said instrument body (1) and is so configured that it aspirates the tissue to the outside surface, and
that said loop (20), that is provided on said electrode array, is disposed laterally on said instrument body at a defined spacing therefrom, and that the shape of said loop (20) is matched with the cross-sectional contour of said outside surface such that the tissue bearing against said outside surface can be removed in layers by a displacement of said loop (20) along the longitudinal axis of said instrument body.

2. Instrument according to Claim 1,
**characterised** in that said outside surface has a cylindrical configuration, and that at least one hole in said outside surface or at least one permeable region constitutes said aspiration element.

3. Instrument according to Claim 2,
**characterised** in that a plurality of holes is provided.

4. Instrument according to any of the Claims 1 to 3,
**characterised** in that said HF electrode array comprises a monopolar electrode (20) that is formed by said moved part.

5. Instrument according to any of the Claims 2 to 3,
**characterised** in that said HF electrode array is a bipolar electrode array including one electrode that is formed by said moved part whilst the other electrode is formed by said cylindrical outside surface area.

6. Instrument according to any of the Claims 1 to 5,
**characterised** in that said loop (20) has the shape of an HF cutting loop known per se and is located at a defined spacing in a direction normal on the surface of said aspiration element, which spacing defines the thickness of the tissue strips to be detached.

7. Instrument according to Claim 6,
**characterised** in that said loop (20) is adapted to be moved over the outside surface area in a direction towards the proximal end for cutting tissue.

8. Instrument according to any of the Claims 2 to 6,
**characterised** in that said instrument body comprises a first hollow shaft (1) which includes said lateral aspiration element (7) and, on its proximal end, a connector (3) for a suction means such as a suction pump.

9. Instrument according to Claim 8,
**characterised** in that said lateral hole array or said permeable region is provided in the outside wall of a hollow body (7) that is inserted into a recess (2) in said first shaft (1).

10. Instrument according to Claim 9,
**characterised** in that said hollow body (7) consists of an electrically insulating material.

11. Instrument according to Claim 10,
**characterised** in that said electrically insulating material is a ceramic material, a glass material, SiO₂ or a synthetic material.

12. Instrument according to Claim 11,
**characterised** in that said hollow body (7) is transparent.

13. Instrument according to any of the Claims 9 to 12,
**characterised** in that said hollow body (7) is inserted into a carrier element (6) together with which it is introduced into said first shaft (1).

14. Instrument according to Claim 13,
**characterised** in that said carrier element (6) is fastened in said first shaft (1) by means of a screw (5) introduced into the distal face of said first shaft.

15. Instrument according to any of the Claims 10 to 14,
**characterised** in that the feed line (18) for said electrode array (20) is disposed outside the axis in said first shaft (1) and is configured as slide rod or tie rod, respectively.

16. Instrument according to any of the Claims 8 to 14,
**characterised** in that a handle (12) is provided on the proximal end of said tie rod (18).

17. Instrument according to Claim 14,
**characterised** in that a manipulator (4) is provided on said first shaft (1).

18. Instrument according to any of the Claims 8 to 17,
**characterised** in that an optical endoscope system (14) is axially introduced into said first shaft (1).

19. Instrument according to Claim 18,
**characterised** in that said optical endoscope system (14) is a lateral-viewing optical system with a field of vision oriented through said transparent hollow body (7).

20. Instrument according to Claim 18,
**characterised** in that said optical endoscope system (14) is a retro optical system and projects beyond the distal end of said first shaft (1).

21. Instrument according to any of the Claims 18 to 20,
**characterised** in that said optical endoscope system (14) is adapted to be axially positioned.

22. Instrument according to Claim 21,
**characterised** in that said optical endoscope system (14) is inserted into a second shaft (8) while it is adapted to be positioned together with this shaft.

23. Instrument according to Claim 22,
**characterised** in that a manipulator (12) such as a thumb ring is attached on said second shaft.

## Revendications

1. Instrument à couper des tissus, comprenant
- un corps de l'instrument allongé (1), apte à être introduit dans le corps humain ou animal,
- un système d'électrodes H.F. (18, 21; 20), auquel on peut appliquer un courant H.F. et qui est déplaçable le long de l'axe longitudinal dudit corps de l'instrument et comprend une boucle (20), et
- un moyen d'aspiration ou respectivement de vide, à appliquer un vide à un élément d'aspiration (7),
**caractérisé** en ce que ledit élément d'aspiration (7) est latéralement disposé dans la surface extérieure dudit corps de l'instrument (1), en étant configuré de façon qu'il aspire le tissu à découper contre la surface extérieure, et
en ce que ladite boucle (20), qui est disposée sur ledit système d'électrodes, est latéralement arrangée sur ledit corps de l'instrument à un écart défini, et en ce que la forme de ladite boucle (20) est adaptée au contour en coupe transversal de ladite surface extérieure d'une manière, que le tissu, qui porte contre ladite surface extérieure, peut être enlevé en couches par un déplacement de ladite boucle (20) le long de l'axe longitudinal dudit corps de l'instrument.

2. Instrument selon la revendication 1,
**caractérisé** en ce que ladite surface extérieure a une configuration cylindrique, et
en ce qu'au moins un trou dans ladite surface extérieure ou au moins une zone perméable constitue ledit élément d'aspiration.

3. Instrument selon la revendication 2,
**caractérisé** en ce qu'une pluralité de trous est formée.

4. Instrument selon une quelconque des revendications 1 à 3,
**caractérisé** en ce que ledit système d'électrodes H.F. comprend une électrode monopolaire (20) constituée par ladite pièce mobile.

5. Instrument selon une quelconque des revendications 2 à 3,
**caractérisé** en ce que ledit système d'électrodes H.F. est un système d'électrodes bipolaire, qui comprend une électrode qui est constituée par ladite pièce mobile, pendant que l'autre électrode est constituée par ladite surface cylindrique de l'enveloppe extérieure.

6. Instrument selon une quelconque des revendications 1 à 5,
**caractérisé** en ce que ladite boucle (20) a la forme d'une boucle de découpage H.F. connue en soi, en se trouvant à un écart défini selon une direction orthogonale sur la surface dudit élément d'aspiration, cet écart définissant l'épaisseur des bandes de tissu à détacher.

7. Instrument selon la revendication 6,
**caractérisé** en ce que ladite boucle (20) est apte à se déplacer sur la surface de l'enveloppe extérieure en une direction vers l'extrémité proximale pour le coupage des tissus.

8. Instrument selon une quelconque des revendications 2 à 6,
**caractérisé** en ce que ledit corps de l'instrument comprend une première tige creuse (1), qui renferme ledit élément d'aspiration (7) latéral et, à son extrémité proximale, un raccord (3) pour un moyen d'aspiration comme une pompe d'aspiration.

9. Instrument selon la revendication 8,
**caractérisé** en ce que ledit système de trous latéral ou ladite zone perméable est disposé dans la paroi extérieure d'un corps creux (7), qui est introduit dans un évidement (2) dans ladite première tige (1).

10. Instrument selon la revendication 9,
**caractérisé** en ce que ledit corps creux (7) est fait en un matériau électriquement isolant.

11. Instrument selon la revendication 10,
**caractérisé** en ce que ledit matériau électriquement isolant est une matière céramique, en verre, en SiO₂, une matière synthétique.

12. Instrument selon la revendication 11,
**caractérisé** en ce que ledit corps creux (7) est transparent.

13. Instrument selon une quelconque des revendications 9 à 12,
**caractérisé** en ce que ledit corps creux (7) est inséré dans un élément porteur (6), ensemble avec lequel il est introduit dans ladite première tige (1).

14. Instrument selon la revendication 13,
**caractérisé** en ce que ledit élément porteur (6) est fixé dans ladite première tige (1) moyennant une vis (5) introduite dans la face distale de ladite première tige.

15. Instrument selon une quelconque des revendications 10 à 14,
**caractérisé** en ce que la ligne d'alimentation (18) pour ledit système d'électrodes (20) est disposée en dehors de l'axe dans ladite première tige (1), en étant configurée sous forme d'une barre de poussé ou respectivement de traction.

16. Instrument selon une quelconque des revendications 8 à 14,
**caractérisé** en ce qu'une manette (12) est disposée sur l'extrémité proximale de ladite barre de traction (18).

17. Instrument selon la revendication 14,
**caractérisé** en ce qu'un manipulateur (4) est disposé sur ladite première tige (1).

18. Instrument selon une quelconque des revendications 8 à 17,
**caractérisé** en ce qu'un objectif endoscopique (14) est axialement introduit dans ladite première tige (1).

19. Instrument selon la revendication 18,
**caractérisé** en ce que ledit objectif endoscopique (14) est un objectif à vue latérale, à un champ des vision orienté à travers ledit corps creux transparent (7).

20. Instrument selon la revendication 18,
**caractérisé** en ce que ledit objectif endoscopique (14) est un objectif du type rétro et fait sailli de l'extrémité distale de ladite première tige (1).

21. Instrument selon une quelconque des revendications 18 to 20,
**caractérisé** en ce que ledit objectif endoscopique (14) est apte à être positionné axialement.

22. Instrument selon la revendication 21,
**caractérisé** en ce que ledit objectif endoscopique (14) est introduit dans une deuxième tige (8), pendant qu'il est apte à être positionné ensemble avec cette tige.

23. Instrument selon la revendication 22,
**caractérisé** en ce qu'un manipulateur (12) comme un anneau repose-pouce, par exemple, est fixé sur ladite deuxième tige.
